(19) Europäisches Patentamt / European Patent Office / Office européen des brevets

(11) **EP 4 144 345 A1**

(12) **EUROPEAN PATENT APPLICATION**

(43) Date of publication:
**08.03.2023 Bulletin 2023/10**

(21) Application number: **21306215.1**

(22) Date of filing: **06.09.2021**

(51) International Patent Classification (IPC):
*A61K 8/9789* (2017.01)    *A61K 8/9783* (2017.01)
*A61Q 19/00* (2006.01)    *A61Q 7/00* (2006.01)
*A61P 17/14* (2006.01)    *A61K 31/00* (2006.01)
*A61K 8/34* (2006.01)    *A61K 8/36* (2006.01)

(52) Cooperative Patent Classification (CPC):
**A61K 8/9789; A61K 8/347; A61K 8/36;
A61K 8/9783; A61K 36/536; A61P 17/14;
A61Q 7/00; A61Q 19/00**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Clariant International Ltd**
**4132 Muttenz (CH)**

(72) Inventors:
• **FRECHET, Mathilde**
 **31400 Toulouse (FR)**

• **CHAJRA, Hanane**
 **31170 Tournefeuilles (FR)**
• **GARANDEAU, David**
 **31390 Lafitte Vigordane (FR)**
• **ROH, Kyung Baeg**
 **16682 Suwon-si (KR)**
• **JUNG, Eun Sun**
 **16533 Suwon-si (KR)**
• **PARK, Deok Hoon**
 **13555 Seongnam-si (KR)**

(74) Representative: **Jacobi, Carola**
**Clariant Produkte (Deutschland) GmbH
Patent & License Management Chemicals
Industriepark Höchst, G 860
65926 Frankfurt (DE)**

(54) **PRUNELLA VULGARIS EXTRACT AND USE THEREOF**

(57) The present invention relates to an extract of the aerial parts of *Prunella vulgaris* targeting the psycho-emotional stress that is responsible for the aggravation of the skin and the hair conditions. Preferably, the present invention relates to the use of a composition comprising or consisting of the aerial parts of *Prunella vulgaris* intended for preventing and/or treating the psychoemotional stress related disorders and/or damages on the skin and the hair follicles.

EP 4 144 345 A1

**Description**

Field of the invention

The present invention relates to the use of an extract of Prunella vulgaris for targeting the psychoemotional stress that is responsible for the aggravation of the skin and the hair conditions. Particularly, the present invention relates to a cosmetic use of a composition comprising at least one extract of the aerial parts of *Prunella vulgaris* intended for counteracting or alleviating the stress-induced sensory neuron's excitation, for regulating the secretion of at least one well-being hormone, for potentializing the release of at least one well-being hormone, or for two or more thereof. Furthermore, the present invention refers to a cosmetic or pharmaceutic composition comprising such extract as an anti-stress active ingredient for hair care and for skin care.

Background of the invention

Stress is a term referring to the body adaptive response to adapt to a perturbation or challenge. It is a state of real or perceived threat to homeostasis, either acute such as an assault or chronic, and can be physiological or psychological.

Psychoemotional stress (PES), arising when people are under mental or emotional pressure, is closely controlled in the brain by the hypothalamic-pituitary-adrenal (HPA) axis that mediates the production of stress response mediators such as cortisol, nerve growth factor (NGF), histamine and substance-P (SP).

Upon sensing stress, the hypothalamus' neurons secrete corticotropin-releasing hormone (CRH), which is transported to the pituitary gland, where it binds to the CRH receptor and stimulates the secretion of proopiomelanocortin (POMC)-derived neuropeptides, including $\alpha$-melanocyte stimulating hormone ($\alpha$-MSH), $\beta$-endorphin, and adrenocorticotropin (ACTH). In turn, ACTH is transported travels in adrenal cortex through the bloodstream and stimulates the production of glucocorticoids (GC) including cortisol, corticosterone, and histamine.

Cortisol is the primary stress hormone in human that regulates a wide range of stress responses. Moreover, when the body perceives stimuli as a threat, the hypothalamus activates the HPA axis and the release of cortisol allows the body to continue to stay on high alert.

Stressful events are inevitable in daily life, but important differences are highlighted between acute and chronic stresses.

Under acute stress, the HPA axis is tightly regulated through feedback mechanisms. Glucocorticoid feedback inhibition plays a prominent role in regulating the magnitude and duration of glucocorticoids release. Acute stress can induce a significant redistribution of lymphocytes from the blood to the skin, leading to enhanced skin immunity and successful stress adaptation. Cortisol can regulate control blood sugar levels, or it can regulate metabolism as well. These functions make cortisol a crucial hormone to protect overall health, to provide adaptative function, and to promote survival or to motivate to success.

In contrast to an acute stress, a chronic stress has no physiological interest. The long-term activation of the stress-response system and the overexposure to cortisol and other stress hormones that follows, can disrupt almost all the body's processes.

As a result, this situation puts the body at increased risk of many health problems, including weight gain, acne, thinning skin, flushed face, sleep problems, severe fatigue, etc.

The skin, the scalp and the hair follicle also developed their own functional peripheral HPA systems where CRH, ACTH, and their receptors are produced within skin cells and hair follicles.

Intracutaneously, the most pronounced endocrine and neuroendocrine activity appears to be located in the skin's major appendages such as the hair follicles (HF) which have become recognized as critically important, independent peripheral (neuro-) endocrine organ.

As the skin is highly innervated, the peripheral cutaneous nerve fibers can impact skin health through secreted factors like neuropeptides (e.g: SP) and neurotrophins (e.g: NGF). They serve as local stress responders that mediate neurogenic inflammation also known as a negative biological cascade. NGF contributes to stress induced cutaneous hyperinnervation, promotes the neurogenic inflammation and affects all hallmarks of allergic inflammation and cutaneous stress responsiveness upstream of SP.

PES increases the production of NGF and glucocorticoids by hair follicles and by skin cells. This exacerbate synthesis induces either the mast cells degranulation and/or the sensory neuron's excitation. In turn, sensory neurons secrete substance-P and neurotrophins such as calcitonin gene related peptide (CGRP) and NGF which reinforce the mast cells activation and, subsequently, the release of inflammatory molecules. Proteases such as tryptase, secreted by activated mast cells, induces the release of inflammatory neuropeptides, such as SP, via PAR-dependent pathways, resulting in an autocrine and/or paracrine vicious cycle mechanism of mast-cell activation with subsequent NGF production.

Several studies and clinical observations highlighted the negative impact of PES on hair growth and stress-induced alopecia. Among them, several models of stress demonstrated the negative impact on the viability and the proliferation of the hair cells and the growth of the hair follicles. In fact, SP and NGF are recruited as key mediators of stress-induced hair growth-inhibitory effects.

In mice for example, it has been demonstrated that the cortisol stress hormone equivalence in rodent regulates the hair follicle stem cell (HFSC) quiescence and the hair growth. Corticosterone, a systemic inhibitor of HFSC activity, was demonstrated to maintain hair follicles in an extended resting phase, and removing such inhibition drives these cells into frequent regeneration cycle a perquisite for hair growth.

Another study evidenced that stress provokes the acceleration of hair greying. Indeed, it was demonstrated, by using animals exposed to different stressors, the part of stress-related neurotransmitters released by nerves hyperactivation on the depletion of melanocytes stem cells and the hair greying.

It is also known that hair follicle cycling is associated with prominent changes in skin perfusion. Also, the epithelial hair bulbs of anagen follicles display angiogenic properties, and the follicular dermal papilla can produce angiogenic factors. The HF-associated blood vascular system undergoes a massive expansion during the anagen phase and a rapid involution during the catagen regression phase. Indeed, studies demonstrated that the production of vascular endothelial growth factor (VEGF)-A is up-regulated during the anagen and its blockage during anti-angiogenic therapy, resulted in thinner HFs.

The above teachings demonstrated that PES causes stress-induced alopecia and hair greying. The over-synthesis of glucocorticoids in skin and its appendages, the over activation of peripheral cutaneous neurons as well as the increase of cutaneous inflammation through mast cells activation, act together in a vicious cycle inducing a hair growth arrest and prolonged resting phase or a hair loss. Moreover, the peripheral cutaneous nerve fibers hyperactivation is involved in the melanin content decrease in hair follicles.

In the peripheral nervous system (PNS), the neurotransmitters beta-endorphins produce analgesia by binding to opioid receptors (particularly of the mu subtype) at both pre- and post- synaptic nerve terminals. When bound, a cascade of interactions results in inhibition of the release of SP, which is involved in the transmission of pain. Thanks to its potent analgesic ability, beta-endorphin is a regulator of physical and psychological stress and plays a role in the modulation of mood, emotion, and behavior. Moreover, the hair follicles are considered as a target and source of neurohormones and neuropeptides, in particular for beta-endorphin. Moreover, beta-endorphin when synthesized within the hair follicles (in hair matrix and hair follicles melanocytes) regulates hair follicles melanocytes proliferation and melanogenesis (Paus et al. 2014, Review CellPress, Vol. 20, Issue 10, P559-570).

Thus, stimulating beta-endorphin production in hair follicles might prevent premature hair greying.

Known for its role in milk let-down and uterine contraction during labor, oxytocin has shown implications in physiological, behavioral, antistress, antidepressant, sociability, and well-being. The cellular response to oxytocin includes regulation of neurite outgrowth, cellular viability, and increased cell survival. Moreover, the oxytocin system is considered as a novel neuroendocrine mediator in human skin homoeostasis and clinically relevant to stressed skin condition.

It has been revealed that both CRF and oxytocin are secreted from the same brain region that regulates the HPA axis and that plasma oxytocin levels correlate positively with the integrity of the HPA feedback mechanism. Accordingly, it has been proposed that cortisol-induced increases in oxytocin may be involved in the feedback regulation of the HPA axis and that there may be important interactions between cortisol-mediated stress and oxytocin-mediated attachment (Tops M. et al., 2012, Frontiers in Psychiatry, Vol. 3, Article 43).

*Prunella vulgaris* is a plant belonging to the family Lamiaceae. It is a perennial herbaceous and a cosmopolitan plant that grows in various parts of the world. It is known to be used for example as an antiseptic, anti-diuretic, and an anti-inflammatory agent.

Extracts from *Prunella vulgaris* are known in cosmetic field. Indeed, JPS-A 6293217 discloses saponins derived from the flower extract of *Prunella vulgaris* as hair tonic agent by inhibiting specifically the reductase activity. Saponins belong to the heterogeneous of glycosides.

X-J Gu et al. 2007, Helvetica Chimica Acta, Vol. 90 (P.72-78) teaches that ethanolic or methanolic extract of the spikes of the flowers of *Prunella vulgaris* contains saponins.

US-A 5624673 teaches *Prunella vulgaris* extract or its saponins content as an active ingredient having the following activities: anti-inflammatory, anti-allergic, epidermis anti-ageing by regulating the renewal and differentiation of keratinocytes and anti-hair loss by promoting the hair follicles renewal. The said extract is obtained at the end of the primary extraction by using solvents selected from water, alcohols (C1 to C4 carbons) or a mixture thereof. The said primary extract contains saponins. Indeed, it is described that a mixture of saponins is obtained from the first aforementioned extract by extracting with an apolar solvent (ether or ketone) which is miscible with the primary solvent. Examples showed formulations comprising a methanolic extract of the leaves of *Prunella vulgaris* for preventing the age-related hair loss. However, this document is silent about the stress-related damages and/or disorders in both skin and hair.

JPH-A 0710722 teaches a hair-growing agent consisting of the ethanolic extract of spikes of *Prunella asiatica* Nakai containing α-amyrin triterpene.

KR-B 101864720 teaches *Prunella vulgaris* extract for preventing the skin atrophy by inhibiting specifically the activity of the enzyme 11β-HSD1, and then inhibiting the production of cortisol. The extract is obtainable by using water, alcohols (C1-C4) or a mixture thereof. However, the document is silent about the part of the plant used. In addition, an aqueous extract of the aerial parts of *Prunella vulgaris* inhibits the expression of the enzyme 11β-HSD1 which catalyzes the intracellular conversion of inactive cortisone into active cortisol. (K-B Roh at al., 2018, Research Article, Article ID 1762478). The said extract also inhibits the cortisone-mediated decrease of collagen content for maintaining the skin integrity and its homeostasis, for preventing the skin atrophy. Lastly, although it is mentioned that rosmarinic acid, contained in the said extract, showed independent inhibitory activity of 11β-HSD1, further active components identification studies is still needed to learn about cortisol-associated skin disease.

The aforementioned extracts of *Prunella vulgaris* have several technical drawbacks. The achieved cosmetic or pharmaceutical beneficial effects are mainly due to the saponins they are containing, needing further extraction steps and additional resources. On the other hand, while the primary extract of *Prunella vulgaris* is used, the related benefit effects are only either for the skin or for the hair, not for both simultaneously. Furthermore, none of the above extract or *Prunella vulgaris* dealt with the psychoemotional stress drawbacks affecting simultaneously, at least partially, the health or the condition of the skin and the hair.

It appears clearly that, under stress condition, regulating the neuro-immune excitation and its secretions, especially of well-being hormones and immune cells, may be a relevant means to prevent or to limit the cutaneous inflammation, particularly by controlling the mast cells degranulation.

Therefore, there is still an unmet need, by the means of a plant extract, to mitigate at least the stress-induced sensitive neurons excitation and/or the neurogenic inflammation, within the skin cells and the skin's major appendages such as the hair follicle cells, for obtaining a well-being sensation by promoting the release of at least one well-being hormone.

There is also another unmet need, by the means of a plant extract, to mitigate the stress-induced sensitive neurons excitation and/or the neurogenic inflammation, more generally to mitigate the psychoemotional stress related disorders and/or damages on the skin and the hair follicles.

These objectives are achieved by using an extract of the aerial parts of *Prunella vulgaris* to protect skin and hair follicles against the psychoemotional stress drawbacks. Especially the use is for protecting the skin against cutaneous inflammation and/or neurogenic inflammation, for providing a well-being and destressing sensation or a soothing effect in a subject, or for two or more thereof. Also, the use is for protecting the hair follicles against stress-induced hair greying, for limiting the stress-induced hair loss, for limiting the stress-induced hair growth arrest, for limiting the stress-induced hair thinning or rarefication, or for two or more thereof.

Summary of the invention

Surprisingly, it has been found that extracting the aerial parts of *Prunella vulgaris* with a convenient solvent can lead to an extract having particular properties for mitigating the psychoemotional stress related disorders and/or damages in both skin and hair and thus, more generally for improving both the skin and the hair conditions.

Accordingly, the present invention relates to an extract of the aerial parts of *Prunella vulgaris* characterized in that:

　　i) it is an aqueous and/or a hydroalcoholic extract of the leaves, stems and flowers;
　　ii) it comprises 1 to 15%, preferably 2 to 12%, more preferably 3 to 10% by weight of one or more polyphenols, based on the total weight of the dry content of the extract;
　　iii) it comprises less than 0,1%, preferably less than 0,01%, more preferably less than 0,001%, particularly 0% by weight of saponins, based on the total weight of the dry content of the extract;
　　iv) a combination of two or more thereof.

Accordingly, the present invention also refers to a method for preparing an extract of the aerial parts (optionally dried) comprising the leaves, stems and flowers of *Prunella vulgaris* comprising the steps of:

　　i) conducting the extraction of the said aerial parts with a water or a hydroalcoholic solvent, wherein:

　　　　a. the water extraction is being performed at 80 to 150°C, preferably at 100 to 130°C, even more preferably at 85 to 130°C, particularly at 90 to 120°C for 15 minutes to 10 hours, preferably for 30 minutes to 8 hours, more preferably for 1 hour to 6 hours, most preferably for 1.5 to 5 hours; or
　　　　b. the hydroalcoholic extraction is conducted at 50 to 120°C, preferably at 60 to 115°C, more preferably at 70 to 110°C, particularly at 80 to 105°C for 15 minutes to 10 hours, preferably for 30 minutes to 8 hours, more preferably for 1 hour to 6 hours, most preferably for 1.5 to 5 hours;

ii) separating the extract from the solid residuals of step (i); and
iii) optionally concentrating the extract by removing the at least one solvent or parts thereof.

The present invention also relates to the (optionally cosmetic) use of a composition comprising (or consisting of) an extract of the aerial parts of *Prunella vulgaris,* as described above, as an active ingredient intended for preventing and/or treating the psychoemotional stress related disorders and/or damages on the skin and the hair follicles. Preferably, the use is for ameliorating the skin and hair conditions subjected to psychoemotional stress.

Accordingly, the present invention also refers to a cosmetic or pharmaceutic composition comprising:

(A) an extract of the aerial parts of *Prunella vulgaris,* as described above, as an active ingredient; and
(B) at least one further cosmetically and/or pharmaceutically acceptable ingredient other than the extract of *Prunella vulgaris,*

wherein the composition is a composition for topic use selected from the group consisting of a solution, a suspension, an emulsion, a cream, a paste, a gel, a lotion, a powder, a soap, a surfactant-containing water, an oil, a shampooing, and a spray; and/or wherein the composition is a nutraceutical composition which is administered orally.

The present invention also relates to a composition comprising (or consisting of) an extract of the aerial parts of *Prunella vulgaris,* as described above, as an anti-stress active ingredient for use in a method for treating or preventing the psychoemotional stress related disorders and/or damages on the skin and the hair follicles.

A further aspect of the present invention refers to a cosmetic method for preventing and/or for treating the stress-induced disorders and/or damages in the skin and in the hair follicles of a subject by applying, onto the skin or the scalp on need, an effective amount of a composition comprising (or consisting of) an extract of the aerial parts of *Prunella vulgaris* as described above.

Brief description of figures

Figures 1A and 1B show the results of measuring the effect of *Prunella vulgaris* extract of the present invention on the release of calcitonin gene related peptide (CGRP) by sensory neurons following cortisol challenge (the stress hormone), respectively after 2H and 24H. Figures 1C and 1D show the quantitative of the effect of *Prunella vulgaris* extract of the present invention on the release of CGRP by sensory neurons following histamine challenge, respectively after 2H and 24H.

In Figures 2A and 2B, it was measured the effect of *Prunella vulgaris* extract of the present invention on the release of oxytocin by the sensory neurons following respectively cortisol and histamine challenges after 2H.

Figure 3 shows the result measuring the proliferation of the hair follicles dermal papilla cells (HFDPCs), in presence of *Prunella vulgaris* extract of the present invention, treated or not with a psychoemotional stress molecule (cortisone).

Figure 4 shows the results of measuring the vascular endothelial growth factor (VEGF) secretion by HFDPC, in presence of *Prunella vulgaris* extract of the present invention, treated or not with cortisone.

Figure 5 shows the enhancing effects of *Prunella vulgaris* extract of the present invention on the human hair follicles growth against cortisol over time.

Figure 6 shows the results of measuring the visible effect of *Prunella vulgaris* extract of the present invention on the hair follicles structure in absence or under cortisol challenge.

Figure 7 shows the results of measuring the effect of a *Prunella vulgaris* extract of the present invention on the number of proliferative or apoptotic hair cells following cortisol challenge.

Figures 8 shows the results of measuring the visible effect of *Prunella vulgaris* extract of the present invention on the hair follicle pigmentation under basal (without stress condition) or cortisol challenge.

Figure 9 shows the results of quantifying the effect of a *Prunella vulgaris* extract on the beta-hexosaminidase activity by basophilic cells (RBL-2H3).

Figures 10A to 10D show the clinical results of measuring the visible effect of *Prunella vulgaris* extract of the present invention for 84 days on the stressed volunteers having stress-induced hair loss. Figure 10A shows the length of the hair follicles over time. Figure 10B shows the proportion of hair follicles in anagen phase over time. Figure 10C shows the proportion of the hair follicles in telogen phase over time. Figure 10D shows the anagen/telogen ratio of the hair follicles over time.

Detailed description of the invention

A first aspect of the present invention relates to an extract of the aerial parts of *Prunella vulgaris* characterized in that:

i) it is an aqueous and/or a hydroalcoholic extract of the leaves, stems and flowers;
ii) it comprises 1 to 15%, preferably 2 to 12%, more preferably 3 to 10% by weight of one or more polyphenols, based on the total weight of the dry content of the extract;
iii) it comprises less than 0,1%, preferably less than 0,01%, more preferably less than 0,001%, particularly 0% by weight of saponins, based on the total weight of the dry content of the extract;
iv) a combination of two or more thereof.

It will be understood that, in a composition consisting of an extract of *Prunella vulgaris,* the composition is the extract.

As used throughout the present disclosure, the extract of the aerial parts of *Prunella vulgaris* of the present invention is also designated as "the extract" or by its abbreviation "PVE" for *Prunella vulgaris* extract.

As used herein, the term "aerial parts" may be understood in the broadest sense as generally understood in the art. It may be constituted by the leaves, the stems, the flowers, which may contain spikes.

As used herein, the term "hydroalcoholic" may be understood in the broadest sense as generally understood in the art. It may be any composition comprising one or more alcohols and water. A hydroalcoholic solvent may be any alcohol/water mixture. In an embodiment, the solvent is an alcohol/water mixture containing between 10 and 90% (v/v) alcohol, or containing between 25 and 85% (v/v) alcohol, or containing between 50 and 82% (v/v) alcohol, or containing between 60 and 80% (v/v) alcohol, or containing between 65 and 75% (v/v) alcohol, or containing approximately 70% (v/v) alcohol.

The quantification of the total polyphenols may be conducted by any means. In a preferred embodiment, the quantification of the total polyphenols content of the extract of the present invention may be performed according to the known Folin-Ciocalteu method. Examples of the polyphenols in the extract of the present invention may be, for example, the caffeic acid and rosmarinic acid.

In an embodiment, the dry content of the extract may represent at least 10 g/L (weight/volume of solvent), preferably at least 15 g/L, even more preferably at least 20 g/L. The solids content is measured by passing the oven at 105°C in the presence of a sand of a sample of given initial weight until a constant weight is obtained.

The extract according to the present invention may be liquid, pasty or solid at standard conditions (room temperature of approximately 20°C, normal pressure of approximately 1013 hPa). In this context, the terms "liquid" and "fluid" may be understood interchangeably. Preferably, the extract is first obtained in liquid form. Then, the one or more solvents may optionally be removed to obtain a solid form of the extract. A liquid extract can also be concentrated under reduced pressure, using but not limited to a vacuum concentrator or a rotary vacuum evaporator. Additionally, after the concentration, the extract may be dried. Then, preferably, parts of the one or more solvents are removed. Optionally, a liquid or pasty extract may be obtained. Optionally, the extract may also be re-diluted with one or more solvents for obtaining a solvent-based extract. Optionally, the extract may also be purified by any means (e.g., crystallization, chromatographic means, etc.).

An extract may be optionally designated as "tincture" or "absolute". The solvent used for the preparation of the solvent-based extract may be an aqueous solvent (water or a buffer), or it may be a combination of an aqueous solvent and a liquid organic solvent. Accordingly, the extract of the aerial parts of *Prunella vulgaris* according to the present invention is a solvent-based extract wherein the dilution solvent may be selected from water, aqueous buffer, alcohols, glycols, ethyl lactate, isopropyl myristate, triglycerides, tri-ethyl citrate, dicaprylyl ether, glyceryl isostearate, glyceryl stearate, ethyl acetate or a mixture thereof. The alcohols may be preferably selected from methanol, ethanol and isopropanol. The glycols may be preferably selected from pentylene glycol and glycerol.

In a preferred embodiment the extract of *Prunella vulgaris* according to the invention is a mixture of glycerol:water in a volume ratio from 95/5 to 5/95, preferably from 80/20 to 20/80.

The extract according to the invention of *Prunella vulgaris* may be optionally obtained in a fraction produced by conducting other purification methods including separation through an ultrafiltration membrane with a constant molecular weight cutoff value, or separation through various chromatographic methods, or liquid-liquid separation or crystallization or precipitation.

As used herein, the term "dry extract" may be understood in the broadest sense as an extract according to the present invention as dry matter, i.e., without the solvent. The dry extract may optionally be physically present as dry matter. It will, however, be directly understood that, in calculations referring a percentage referred to dry matter, it does not have to be dry matter present in physical form, but the dry extract may also be present in dissolved form. Then, in the calculation, the mass of the solvent is mathematically subtracted from the total mass.

Dry matter (in physical form) may be obtained by a suitable extraction method, followed by a step of drying the extract obtained. The drying may be performed by any method suitable for this purpose known in the art. Drying means removing the one or more solvents used for extraction. Removing the one or more solvents may be performed by any means. For example, it may be achieved by subjecting the extract to a hot and dry atmosphere and/or placing the extract on a heated plate in order to evaporate the solvent. For example, drying may be achieved by evaporation in a vacuum and/or at elevated temperature (e.g., in a rotary (vacuum) evaporator) or by crystallization of the solid material of interest. Alternatively, or additionally, drying may be achieved by out by atomisation or by lyophilization.

In an embodiment of the invention, the extraction solvent may be selected from the group comprising a water and a hydroalcoholic solution. Alcohols may be selected from ethanol, methanol, propanol, butanol, pentanol, phenol, glycerol, 1,3-butylene glycol, propane diol, or a mixture of two or more thereof. Thus, the extract may be in the form of aqueous and/or hydroalcoholic extract. In a preferred embodiment of the invention, the solvent is a water and/or a hydroalcoholic (for example: hydromethanolic and/or hydroethanolic). In a particular embodiment, the solvent is water, then the extract is an aqueous extract.

As used herein, the term "extract" may be any substance made by extracting the leaves, stems and flowers of *Prunella vulgaris.* In accordance with the present disclosure, the PVE is a solvent-based extract obtained by solvent extraction of the said plant. As used herein, extracting is achieved by using at least one solvent, preferably a polar solvent. Accordingly, in a preferred embodiment, the extract is obtained by performing a solvent extraction method. In a preferred embodiment, the extract is obtained from aqueous and/or hydroalcoholic extraction methods including cold or hot extraction, ultrasonic extraction, reflux cooling, needle extraction and microwaves extraction. However, the solvent used as well as the method are not limited thereto as long as an extract providing the inherent benefit effects in accordance with the spirit of the present disclosure may be obtained. The solvent may be added to the plant material. This may be achieved by any means known in the field such as, e.g., filtration, sieving, ultrafiltration, cross-flow filtration, centrifugation, precipitation over time, or a combination of two or more thereof.

In a preferred embodiment, the said extract of *Prunella vulgaris* is an aqueous extract.

In an embodiment, the hydroalcoholic extract of *Prunella vulgaris* is a hydromethanolic and/or a hydroethanolic extract.

An aspect of the present invention relates to a method for preparing an extract of the aerial parts (optionally dried) comprising the leaves, stems and flowers of *Prunella vulgaris* comprising the steps of:

i) conducting the extraction of the said aerial parts with a water or a hydroalcoholic solvent, wherein:

a. the water extraction is being performed at 80 to 150°C, preferably at 100 to 130°C, even more preferably at 85 to 130°C, particularly at 90 to 120°C for 15 minutes to 10 hours, preferably for 30 minutes to 8 hours, more preferably for 1 hour to 6 hours, most preferably for 1.5 to 5 hours; or

b. the hydroalcoholic extraction is conducted at 50 to 120°C, preferably at 60 to 115°C, more preferably at 70 to 110°C, particularly at 80 to 105°C for 15 minutes to 10 hours, preferably for 30 minutes to 8 hours, more preferably for 1 hour to 6 hours, most preferably for 1.5 to 5 hours;

(i) separating the extract from the solid residuals of step (ii); and
(ii) optionally concentrating the extract by removing the at least one solvent or parts thereof.

In one specific embodiment, the hot-water extraction is performed on the dried powder of the aerial parts in purified water at high temperature, typically at 80 to 150°C, preferably at 100 to 130°C, even more preferably at 85 to 130°C, particularly at 90 to 120°C for 15 minutes to 10 hours, preferably for 30 minutes to 8 hours, more preferably for 1 hour to 6 hours, most preferably for 1.5 to 5 hours, the extraction may be conducted under reflux. The ratio of the plant material to the extraction solvent may be comprised between 1:10 and 1:40, preferably between 1:15 and 1:35, more preferably between 1:20 and 1:30 by weight. The liquid obtained may be filtered, concentrated under reduced pressure at the temperature between 20 to 60°C, preferably between 25 to 55°C and then spray dried.

In an embodiment, extracting is conducted at ambient pressure (i.e., 980 to 1200 hPa), at hot temperature (i.e., 80 to 120°C) using water and/or an alcohol/water mixture containing between 5 and 95% (v/v) alcohol.

In the context of the invention, the extract is preferably either dried, or frozen or freeze-dried, or fresh. The plant material may be used in any form such as, e.g., as powder, crushed or any other form.

The extract according to the present invention may be obtained by any means. It may optionally be obtained by a commercial supplier or may be partly or completely prepared from the aerial parts of *Prunella vulgaris*. In a preferred embodiment, the solvent is water, an aqueous buffer or a hydroalcoholic. In a particular preferred embodiment, the solvent is water and the extraction method is conducted by hot water extraction during a suitable time.

The hydroalcoholic solvent is a solvent mixture that is mainly composed of one or more alcohols and water. Preferably, the hydroalcoholic solvent is a solvent mixture that is composed of more than 50% by weight, at least 60% by weight, at least 70% by weight, at least 80% by weight, at least 90% by weight, at least 95% by weight, consists (essentially) of one or more alcohols and water. Optionally, the hydroalcoholic solvent is a solvent mixture that is composed of more than 50% by weight, at least 60% by weight, at least 70% by weight, at least 80% by weight, at least 90% by weight, at least 95% by weight, consists (essentially) of one or more alcohols. As used herein, water may also include hot water or subcritical water. Also, one or more aqueous buffers may be used.

Accordingly, an aspect of the present invention refers to an extract of the aerial parts of *Prunella vulgaris* obtainable by implementation of the preparation method as described above.

Another aspect of the present invention relates to the cosmetic use of a composition comprising (or consisting of) an extract of the aerial parts of *Prunella vulgaris* as described above as an active ingredient intended for preventing and/or treating the psychoemotional stress related disorders and/or damages on the skin and the hair follicles.

In an embodiment of the invention, the stress-related disorder and/or damage is selected from skin dryness, skin redness, skin aberrant pigmentation, sebum-deregulation, skin itching, skin inflammation, atopic dermatitis, psoriasis, vitiligo, systemic lupus erythematosus, hair loss (i.e., alopecia areata, telogen effluvium), hair growth arrest, hair follicles apoptosis, hair thickness, hair greying and hair thinning, or two or more thereof.

In a preferred embodiment, the stress-related disorders may be hair loss, hair growth arrest, hair follicles apoptosis, hair thickness, hair greying and hair thinning.

In an embodiment, the extract of the present invention is for regulating the stress-induced neuro-immune excitation, for regulating the stress-induced sensory neuron excitation, for counteracting the stress-induced mast cell degranulation, for promoting the secretion of at least one well-being hormone, or for two or more thereof.

Advantageously, the tissue comprises the skin and the scalp. The skin may be dry skin, healthy skin, normal skin and oily skin.

The extract according to the invention may be used as an active ingredient. Moreover, it may be used as such in pure form or may be mixed with one or more other components. In other words, it may be used as anti-stress active ingredient in a composition, meaning that it is able to limit and/or to fight against the psychoemotional stress drawback effects in the skin and the hair follicles. Preferably, it is used in a cosmetic or a pharmaceutic composition.

Notably, the experimental studies provided in the present disclosure demonstrated that the compositions comprising (or even consisting of) an extract of the aerial parts of *Prunella vulgaris* of the present invention may be used as an anti-stress active ingredient for cosmetic and/or pharmaceutical purposes, in particular for the skin care and the hair care. Indeed, the compositions comprising (or even consisting of) *Prunella vulgaris* may improve both skin and hair conditions, particularly when they have been faced to a psychoemotional stress condition.

It was thus surprisingly found that the extract of the present invention may regulate positively, in response to a psychoemotional stress, at least one or more specific physiological responses leading to inherent beneficial effects on both skin and hair, justifying its use in cosmetic and pharmaceutical purposes.

Notably, the extract according to the present invention enables to trigger the secretion of well-being hormones, for regulating the neuro-immune excitation induced by at least a stress mediator and/or for regulating the release of well-being hormones such as, for example, the beta-endorphin and the oxytocin. Indeed, as explained above, psychoemotional stress through neuro-immune activation increases the production of NGF and glucocorticoids by hair follicles and skin cells. This induces either the mast cells degranulation and/or the sensory neuron excitation, followed by the secretion of substance-P and neurotrophins such as CGRP and NGF which reinforce the mast cells activation and, subsequently, the release of inflammatory molecules. The experimental data demonstrated that the extract according to the present invention is able to counteract or to mitigate the secretion of the at least one the above stress mediators (i.e., cortisol, CGRP, histamine). Hence, the conditions of the skin and the hair may be improved, as well as the well-being sensation may be obtained through the production, for example, of oxytocin, beta-endorphin, dopamine and serotonin.

In an embodiment of the present invention, the well-being hormone may comprise the beta-endorphin, the oxytocin, the serotonin, the dopamine or a combination of two or more thereof.

The stress mediators may comprise the cortisol and like (i.e., cortisone), the histamine, the adrenaline, the noradrenaline and like, or a combination of two or more thereof.

Additionally, the extract of the present invention enables to protect the hair follicles against the stress-related apoptosis and the decrease of hair follicles proliferation. Indeed, glucocorticoid such as cortisol induces a negative impact on the viability and the proliferation of the hair cells and the growth of the hair follicles and its vitality.

Furthermore, the extract according to the present invention enables to counteract the stress-induced alteration of hair follicles and/or for alleviating the cortisol-induced hair growth arrest. Indeed, experimental data demonstrated that the extract according to the present invention enables to prevent the hair follicles apoptosis and to restore the proliferative activity of these cells.

Furthermore, the extract according to the present invention enables to counteract the stress-induced alteration of hair follicles pigmentation responsible for hair greying. Indeed, experimental data demonstrated that the said extract enables to prevent the hair follicles loss of pigmentation observed under stress condition.

In a preferred embodiment of the present invention, the extract may have at least one of the following activities on the skin cells and/or the hair follicles:

> (a) counteracting and/or reducing the stress-induced degranulation of mast cells;
> (b) preventing the skin stress-induced inflammation and/or irritation;
> (c) alleviating or counteracting the cortisol-induced hair growth
> (d) restoring the stressed hair follicles in producing VEGF;
> (e) alleviating or counteracting the cortisol-induced hair loss;
> (f) counteracting the cortisol-induced hair follicles damages;
> (g) promoting cell proliferation reduced in hair follicles exposed to cortisol;
> (h) counteracting the cortisol-induced hair follicles apoptosis;
> (i) preventing or limiting the cortisol-induced hair greying;
> (j) promoting the hair densification reduced by the cortisol;
> (k) preventing or limiting the cortisol induced hair thinning;
> (l) a combination of two of more thereof.

It will be understood that the definitions and preferred embodiments made in the context of the extract of *Prunella vulgaris* of the present invention or a composition comprising such extract of the present invention *mutatis mutandis* apply to the use thereof.

As used throughout the present invention, the terms "psychoemotional stress" or "stress" may be understood interchangeably in the broadest sense as generally understood in the art. In other words, it refers to a body's state arising when people are under mental and emotional pressure, leading to the production, by the hypothalamic-pituitary-adrenal (HPA) axis, of stress mediators including substance P, neurotrophins such as calcitonin gene related peptide (CGRP) and NGF, glucocorticoids such as cortisol, corticosterone, and histamine. For example, these stress mediators may be secreted following, many stressors but not limited to, such as work responsibilities, financial strains, health issues, domestic conflicts can trigger the psychoemotional stress. This stress is often associated with physical symptoms such as headaches, sleep disturbances, digestives issues and hair loss. Altogether, these symptoms negatively impact our well-being. Moreover, light exposure (e.g., exposure to ultra-violet (UV) light, exposure visible light and/or exposure to infra-red (IR) light, exposure to particular pollution, exposure to one or more pathogens (including natural and chemicals products) and/or allergens, exposure to fluctuating temperature and/or humidity, and/or exposure to mechanical constraints (including such due to daily habits such as shaving, gloves, and/or wearing masks) can also stimulate these stress mediators.

As used throughout the present invention, the terms "stress-induced" or "stress related" may be understood interchangeably in the broadest sense as generally understood in the art. In other words, the stress-induced refers to the physiological and physical effects due to the stress on the skin and/or on the hair.

As used herein, the term "well-being hormone" or "happiness hormone" may be understood interchangeably in the broadest sense as generally understood in the art as a molecule (e.g., hormone consisting of proteins or peptides, or may be a chemical molecule) enabling to provide a well-being or a soothing effect in a subject.

As used herein, the term "neuro-immune excitation" refers to the excitation of both the sensory neurons and the immune system leading to the mast cell degranulation and the release of well-being hormones. As used herein, the term "sensory neuron excitation" may be understood in the broadest sense as generally understood in the art. In other words, it means that, after receiving an exciting signal (i.e: at least one stress mediator), the sensory neurons trigger the correspondent responses by mediating the production of stress response mediators such as cortisol, nerve growth factor (NGF), histamine and substance-P (SP), and mediating the production of well-being hormones such as oxytocin and beta-endorphin as well.

As used herein, the term "regulating" refers to the balanced response provided by the neuron depending on the context. Indeed, when a sensory neuron is excited, it leads both to the secretion of at least one well-being hormone and also the production of stress response mediators.

Surprisingly, it has been found that inventive extract can be used as an anti-stress active ingredient.

Accordingly, a further aspect of the present invention relates to a cosmetic or a pharmaceutic composition comprising:

A. an extract of the aerial parts of *Prunella vulgaris,* as described above, as an active ingredient; and
B. at least one further cosmetically and/or pharmaceutically acceptable ingredient other than an extract of *Prunella vulgaris,*

wherein the composition is a composition for topic use selected from the group consisting of a solution, a suspension, an emulsion, a cream, a paste, a gel, a lotion, a powder, a soap, a surfactant-containing water, an oil, a shampooing, and a spray; and/or wherein the composition is a nutraceutical composition which is administered orally.

It will be understood that the definitions and preferred embodiments made in the context of the use of an extract of *Prunella vulgaris* of the present invention *mutatis mutandis* apply to a composition comprising such extract.

Herein, the extract according to the invention may optionally be considered as an (or optionally even the sole) active ingredient. As used herein, the term "active ingredient" may be understood in the broadest sense as a component that may exhibit a desired and intended activity, either alone or together with one or more other ingredients such as one or more carriers that are themselves inactive or other active ingredient that may optionally act synergistically. Preferably, the active ingredient of the present invention is a cosmetically active ingredient. More preferably, the active ingredient of the present invention is an anti-stress related active ingredient. As used herein, the expression "anti-stress" in the context of the present invention means that such active enables to fight against the physical disorders and/or damages caused by stress and it enables to provide a psychological benefit such as relaxation, destressing.

The cosmetic or pharmaceutic composition may be used for any purpose and in any form. In a preferred embodiment, the composition is a composition for topic use selected from the group consisting of a solution, a suspension, an emulsion, a cream, a paste, a gel, a lotion, a powder, a soap, a surfactant-containing water, an oil, a shampooing, and a spray, or wherein the composition is a nutraceutical composition which is administered orally.

The composition may also be or may be comprised in a product selected from the group consisting of emulsions, gels, ointments, tonics, liquid soaps, bar soaps, bath oils, shower oils, massage oils, makeups, scalp treatments, aftershaves, shaving products, deodorants, shower gel, shampoos, and combinations of two or more thereof.

Optionally a cosmetic or pharmaceutical composition may also be a nutraceutical composition which can be administered orally to a subject. Then, the cosmetic or pharmaceutical composition may optionally be included in a food product, such as a food supplement. Then, the composition may typically have a systemic effect. A nutraceutical composition according to the present invention may be formulated, with acceptable carriers, in any form suitable for oral administration such as, e.g., tablets, capsules, granules, powder, solution, emulsion, or suspension.

The cosmetic or pharmaceutic composition may comprise the extract of the present invention in any content. In a preferred embodiment, the composition comprises less than 30% by weight, referred to the total weight of the composition, of an extract of according to the present invention. In a preferred embodiment, the composition comprises 0.0001 to 20% by weight, more preferably 0.001 to 15% by weight, even more preferably 0.005 to 10% by weight, in particular 0.01 to 5% by weight, referred to the total weight of the composition, of the extract according to the present invention.

The at least one further cosmetically and/or pharmaceutically acceptable ingredient other than the extract of the invention may be any cosmetically and/or pharmaceutically acceptable ingredient. In a preferred embodiment, the cosmetically and/or pharmaceutically acceptable ingredient is or comprises at least one cosmetically and/or pharmaceutically acceptable carrier.

As used herein, the terms "pharmaceutically acceptable carrier", "pharmaceutically acceptable excipient", "cosmetically acceptable carrier", "cosmetically acceptable excipient", "carrier" and "excipient" may be understood interchangeably in the broadest sense as any substance that may support the cosmetic and/or pharmacological acceptance or usability of the composition according to the present invention containing the extract of *Prunella vulgaris.* Preferably, neither the extract of *Prunella vulgaris* nor a composition containing such at least one extract according to the present invention is toxic when applied to the tissue.

The composition ready to use preferably may be a liquid formulation, in particular a composition suitable for topic and oral administration. The storage form of the composition may also be liquid, but may also be a dried form (e.g., a powder such as a powder comprising or consisting of an extract of *Prunella vulgaris*), or may be a paste or syrup or the like. Optionally, a dried form, paste or syrup may be dissolved or emulsified prior to being administered to the skin of interest.

A cosmetically and/or pharmaceutically acceptable carrier may exemplarily be selected from the list consisting of an aqueous buffer, saline, water, alcohols, vegetable oils, mineral oils, polymers, or combination of two or more thereof. Optionally, a cosmetically and/or pharmaceutically acceptable carrier may contain one or more cosmetically and/or pharmaceutically acceptable additives.

In a preferred embodiment, such cosmetically and/or pharmaceutically acceptable additives may be selected from the group consisting of fragrances/perfumes, dyes, pigments, emulsifiers, lubricants, chelating agents, acidity regulators, antimicrobial agents, preservatives, antioxidants, and combinations of two or more thereof. For instance, the cosmetically and/or pharmaceutically acceptable carrier may optionally contain one or more detergents, triethanolamine, one or more fragrances, one or more foaming agents (e.g., sodium lauryl sulphate (SLS), sodium doceyl sulphate (SDS)), one or more colouring agents (e.g., food colouring, pigments), one or more vitamins, one or more salts (e.g., sodium, potassium, calcium, zinc salts), one or more humectants (e.g., sorbitol, glycerol, butylene glycol, propylene glycol, mannitol, propylene glycol, polydextrose), one or more enzymes, one or more preserving agents (e.g., benzoic acid, methylparabene, one or more antioxidants, one or more herbal and plant extracts, one or more stabilizing agents, one or more chelating agents (e.g., ethylenediaminetetraacetic acid (EDTA), one or more polymers (e.g., carboxyvinyl polymer, carboxymethyl cellulose, cellulose, carboxyethyl cellulose), one or more stabilizers, one or more solubilizers, one or more emollients, and/or one or more uptake mediators (e.g., polyethylene imine (PEI), a cell-penetrating peptide (CPP), a protein transduction domain (PTD), an antimicrobial peptide, etc.).

Moreover, the composition of the present invention may contain, in addition to the extract of *Prunella vulgaris,* one or more other known active ingredients, working either synergistically and/or complementarily with the said extract of *Prunella vulgaris* without affecting its activity or its indented use as mentioned above.

Examples of known active ingredients may be, but not limited to, those described in the documents EP-A 3681467 and EP-A 3643295, which are herewith incorporated by reference.

The composition according to the present invention may be cosmetic product or may be comprised in a cosmetic product. Stress-related deleterious effects in the skin and hair may be reduced or avoided, according to the safety studies and legal requirements. The composition may be for any use. Any administration routes are suitable that lead to the desired purpose as claimed. Administration may be local or systemic administration. Preferably, administration is local administration. Administration may be topic administration, transdermal administration, oral administration, administration by mean of injection (e.g., intravenous (i.v.), intraarterial (i.a.), intraperitoneal (i.p.), intramuscular (i.m.), and subcutaneous (s.c.) injection), or nasal administration. For example, the composition according to the present invention may be formulated, with acceptable carriers in any suitable form for topical, oral, rectal, transmucosal, transnasal, intestinal, enteral and parenteral administrations.

In a preferred embodiment, the composition is a composition for topic use. In a preferred embodiment, the composition is a composition for topic use which is administered topically to a subject. In a preferred embodiment, the composition is a composition for topic use which is administered topically to a part of the skin such as, e.g., to the face, scalp, or any part of a body. Skin may be all types of skin, including compromised skin, dry skin, healthy skin, normal skin and oily skin.

As indicated above, the extract of *Prunella vulgaris* or the composition of the present invention may be used for any purpose. Preferably, it is used for a cosmetic and/or pharmaceutical purpose.

The extract of the present invention or a composition comprising such extract may have a local and/or a systemic effect. In a preferred embodiment, when administered locally and/or topically, it (mainly or completely) has a local effect.

As used therein, the terms "compromised skin" and "compromised skin phenotype" may be understood in a broad sense as any type of skin that is exposed to one or more harmful factors involved in triggering, at least, a psychoemotional stress.

A subject having the tissue of interest, in particular skin, scalp and hair, may be subjected from or may be of risk of developing a disorder and/or damage as laid out above.

As used in the context of the present invention, the term "subject" may be understood in the broadest sense as any living being. A subject preferably is a human or animal, more preferably a human or mammal, in particular a human being.

As used herein, the term "disorder" may be understood in the broadest sense as any pathological condition. It may preferably be an inherent disease. As used herein, the term "damage" may be understood in the broadest sense as any perturbation or dysfunction in a tissue, particularly in (otherwise) healthy tissue. Particularly, as used herein, the stress-related disorders and stress-related damages may be inherently triggered by factors enabling to induce psychoemotional stress within a tissue. Tissue means preferably the skin, including the scalp, and the hair follicles.

Throughout this specification and the claims, unless the context requires otherwise, the open-worded terms such as "comprise", "contain", "include", etc., and variations such as "comprises", "contains", "includes", "comprising", "containing", "including", etc., maybe understood to imply the inclusion of a stated component, member, integer or step or group of components, members, integers or steps, but not the exclusion of any other components.

The terms "a" and "an" and "the" and similar reference used in the context of describing the invention (especially in the context of the claims) are to be construed to cover both the singular and the plural, unless otherwise indicated herein or clearly contradicted by the context. Recitation of ranges of values herein is merely intended to serve as a shorthand method of referring individually to each separate value falling within the range. Unless otherwise indicated herein, each individual value is incorporated into the specification as if it were individually as well as in combination with each other recited herein. All methods and procedural steps, including product-by-process steps described herein can be performed in any suitable order unless otherwise indicated herein or otherwise clearly contradicted by context. The use of any and all examples, or exemplary language (e.g., "such as", "for example"), provided herein is intended merely to better illustrate the invention and does not pose a limitation on the scope of the invention otherwise claimed. No language in the specification should be construed as indicating any non-claimed element essential to the practice of the invention.

The present invention also refers to a cosmetic method intended for preventing and/or for treating stress-induced disorders and/or damages in the skin and in the hair follicles, of a subject, by applying, onto the skin or the scalp on need, an effective amount of a composition comprising (or consisting of) an extract of the aerial parts of *Prunella vulgaris* as described above.

It will be understood that the definitions and preferred embodiments made in the context of the use of extract of *Prunella vulgaris* of the present invention or the use of a composition comprising such extract of the present invention *mutatis mutandis* apply to the cosmetic method thereof.

Another aspect of the present invention relates to a composition comprising (or consisting of) an extract of the aerial parts of *Prunella vulgaris* as described above as an active ingredient, particularly as an anti-stress active ingredient, for use in a method for preventing and/or for treating the stress-induced disorders and/or damages in the skin and in the hair follicles.

More generally, it should be understood that the aspects and embodiments may be combined in any manner and in any number to additional embodiments. Any permutations and combinations of all described elements in this application should be considered disclosed by the description of the present application unless the context indicates otherwise.

Unless defined otherwise, all technical and scientific terms used herein have the same meaning as commonly understood by a person skilled in the art. Although the methods and materials described herein are preferred, other methods and materials similar or equivalent to those described herein can be used in the practice or testing of the present invention as well.

All documents cited or referenced herein are hereby incorporated by reference and may be employed in the practice of the invention. More specifically, all referenced documents are incorporated by reference to the same extent as if each individual document was specifically and individually indicated to be incorporated by reference.

The following examples and figures are intended to illustrate further embodiments of the present invention without limiting its scope.

Examples

1. Preparation of extract of *Prunella vulgaris*

Leaves, stems and flowers of *Prunella vulgaris* were collected and cleaned thoroughly to remove the impurities and dried at 40 to 50°C and pulverized to a particle size of 1 mm or less. Then, 100 g of the powder was immersed in 3 L of distilled water. Extraction with a boiling water under reflux was carried out 3 times for 2 hours. The liquid obtained was filtered on a filter paper (Advantec n°2), concentrated under reduced pressure at 50°C and then spray dried for obtaining 13 g of the dried *Prunella vulgaris* extract. 10 g of this dried powder was solubilized in 1 L of a solution of glycerol/water (80/20) (v/v) for obtaining the final so-called *Prunella vulgaris* extract (hereinafter PVE) at 10 g/L. The total polyphenols content of PVE, measured according to the Folin-Ciocalteu method, represented 0.73 g/L or 7,3% by weight, based on the total weight of the dry extract. The liquid chromatography-mass spectrometry (HPLC-MS) analysis performed on the PVE obtained does not reveal the presence of saponins.

The above PVE was used for the subsequent tests.

*2. In-vitro* studies:

2.1. Evaluation of the beta-endorphin like effect of the PVE

To evaluate the effect of PVE on the sensory neuron's excitation under cortisol and histamine challenges, the following experiments were conducted.

Human sensory neurons are derived from hiPS cells (human induced Pluripotent Stem cells). hiPS cells are obtained by transfecting human fibroblasts cells. Cells were plated in 96 wells plates coated by a thin layer of Matrigel® (Corning; ref: 354277; batch: 9238002) in a differentiation medium (M1). Cells were incubated at 37°C and 5% $CO_2$ for 6 days and culture medium was changed every 2 days.

After 9 days of differentiation, medium M1 was replaced by a maturation medium for human sensory neurons (M2). Cells were incubated at 37°C and 5% $CO_2$ for 6 days and culture medium was changed every 2 days. On day 18 of culture (24 hours of pre-incubation) or day 19 of culture (2 hours of pre-incubation,) the medium was removed, and fresh medium with PVE or reference compound β-endorphin (Bachem; ref: H-2700; batch: 1069755) was added. The following conditions were done: control medium, control medium containing PVE at 50, 100 or 150ppm, control medium containing beta-endorphin at 10μM. Culture was incubated for 24 hours at 37°C and 5% of $CO_2$. After 2 or 24 hours of incubation, the culture plates were stimulated by cortisol (Sigma Aldrich; ref: H0888; batch: SLBN5690V) or histamine, each at $10\mu M$ (Tocris; ref: 3545; batch: 1A/18942). After 30 minutes of stimulation, supernatants were removed and stored at -80°C.

The amount of CGRP (antibodies-online; ref: abx257902; batch: E2001551F) released in supernatant after 30 minutes of stimulation were dosed by ELISA and compared to non-treated control. All values were expressed as mean +/- s.e.mean. Statistics analyses were done using an ANOVA assay followed by Dunnett's test. *$p<0.05$; **$p<0.005$ ; *** $p<0,001$ vs control condition. ### $p<0,001$ vs cortisol or histamine condition.

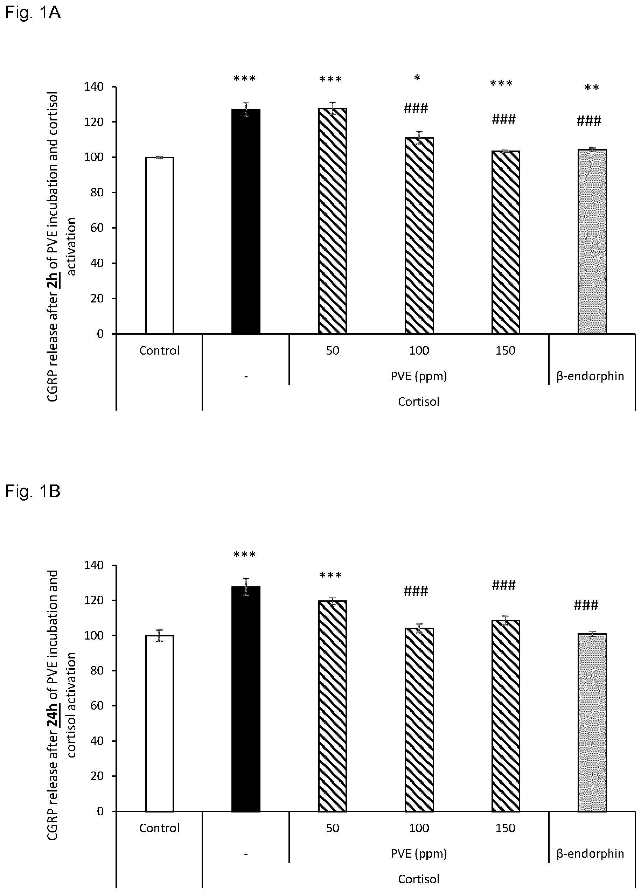

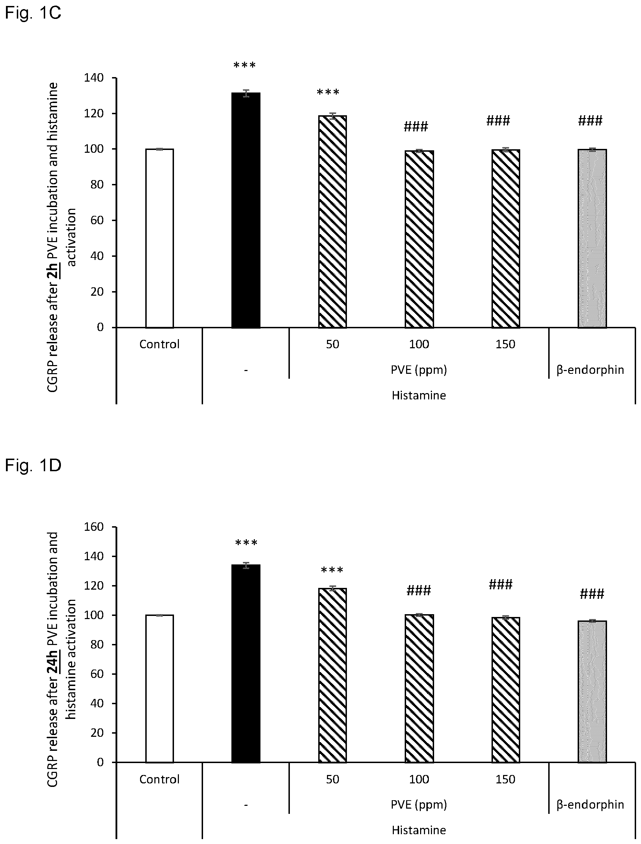

As a result, as shown in FIGS. 1A, 1B, 1C and 1D, both cortisol and histamine challenges significantly increase the sensory neuron's excitation, showing by a strong increase of CGRP release. The results demonstrated that PVE counteracts or alleviates the stress-induced sensory neuron's excitation, either after short (2 hours) or long (24 hours) pre-incubation with stress molecules. Indeed, firstly cortisol induces the sensory neuronal excitation, as confirmed by the increase of CGRP release, and beta-endorphin under stress condition is able to reduce the neuronal excitation at level similar to the control condition. Secondly, when sensory neurons are treated with PVE under a stress condition, it was demonstrated the ability of PVE to mitigate the sensory neuronal excitation which leads to the secretion of SP, CGRP and NGF which altogether reinforces the mast cells activation and, subsequently, the release of inflammatory molecules. Indeed, the level of CGRP is decreased in a dose dependent manner. Interestingly, it was demonstrated that PVE triggers the same well-being effect as beta-endorphin. In FIGS. 1C and 1D, it was demonstrated the ability of PVE to counteract the sensory neuronal excitation induced by histamine. Interestingly, it was demonstrated that PVE triggers the same well-being effect than beta-endorphin.

2.2. Evaluation of the well-being effect of the PVE

To evaluate the effect of PVE on the oxytocin release under cortisol and histamine challenges, the following experiments were conducted.

Based on the previously explained protocol for sensory neurons culture, the amount of Oxytocin (antibodies-online; ref: ABIN2862593; batch: 20XT015a) released in supernatant after 30 minutes of stimulation were dosed by ELISA and compared to non-treated control.

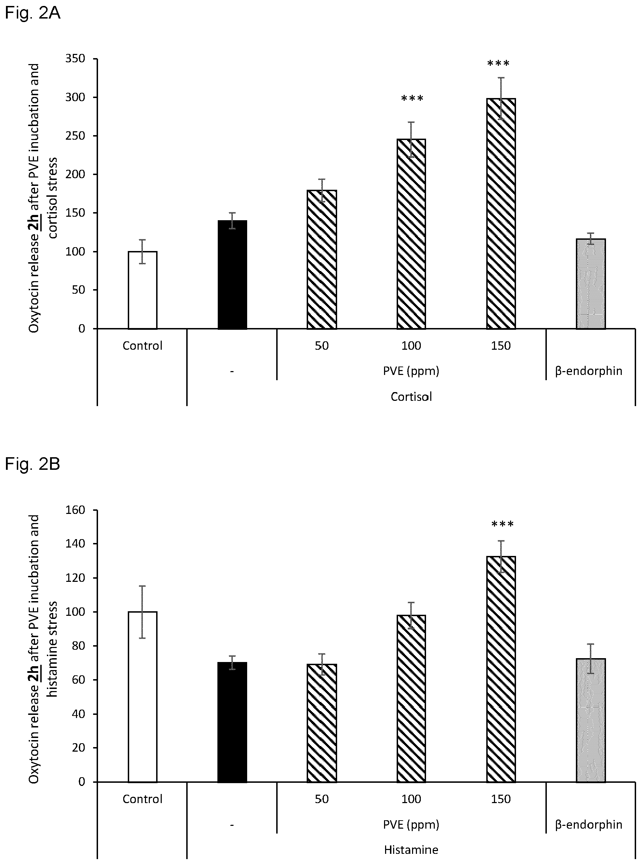

As a result, as shown in FIGS. 2A and 2B, it was confirmed that PVE strongly increased the oxytocin release by the sensory neurons, after 2 hours of pre-incubation. In FIG. 2A it was demonstrated the ability of PVE to promote the secretion of oxytocin (well-being effect) and to counteract the sensory neuron excitation induced by cortisol. In FIG.2B, it was measured the effect of PVE on the release of oxytocin by the sensory neurons following histamine challenge. These results demonstrated the ability of PVE to promote the secretion of oxytocin and to counteract the sensory neuron excitation induced by histamine. Moreover, this experiment showed that beta-endorphin has no effect on the oxytocin release, demonstrating a widest activity of the PVE in response to both cortisol and histamine challenges, meaning in response to stress conditions. In conclusion, these results emphasized the well-being potential ability of the PVE, as it was clearly demonstrated the dose dependent release of oxytocin under both cortisol and histamine stresses conditions.

2.3. Evaluation of the ability of PVE to counteract the glucocorticoids-induced

decrease proliferation of hair follicles cells and to restore the VEGF secretion HFDP cells were maintained in Follicle Dermal Papilla Cell Growth Medium (C-26501, PromoCell, Heidelberg, Germany) at 37°C, under 5%, $CO_2$. Cells at passage 4 were plated in 24 well plate at $2 \times 10^4$ cells/well. HFDPCs at 60% confluence was pretreated with 5, 10 or 20 ppm of PVE for 2 h, and subsequently treated with 200 $\mu M$ cortisone. After incubation for 60 h, cell culture supernatant was recovered, and secreted VEGF was measured using ELISA kit. For the cell proliferation assay, the WST test was used (EZ-Cytox, DogenBio, Korea). Absorbance was measured at 450 nm wavelength using microplate spectrophotometry.

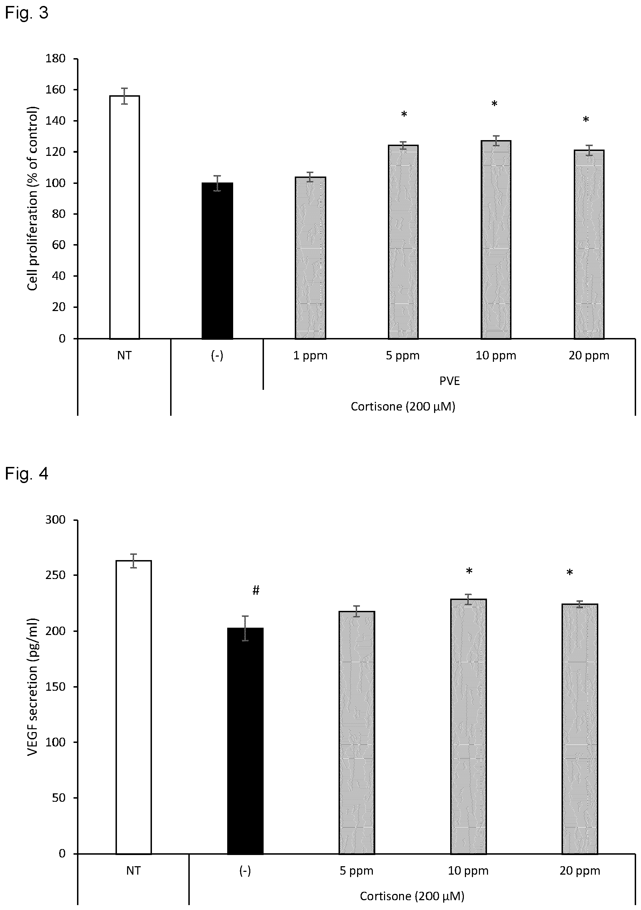

As shown in FIGS.3, as expected, it was demonstrated that the psychoemotional stress leading to cortisone production inhibits the HFDPCs proliferation. These cells are a major player in hair physiology. The results also demonstrated the ability of PVE to restore the cortisone-induced hair growth arrest. Indeed, the results demonstrated the ability of PVE in promoting the growth of HFDPCs despite a cortisone treatment, thus suggesting the beneficial effect of PVE in hair physiology, especially the promotion of hair growth.

As shown in FIG. 4, the result demonstrated the ability, in dose dependent manner, of PVE in restoring the secretion of VEGF under cortisone treatment. Indeed, as expected, it was demonstrated that the psychoemotional stress leading to cortisone production, reduced VEGF production. VEGF is key in the hair physiology as it participate to the regulation of microcirculation around hair follicle thus improving the nutrition of hair follicles. The results demonstrated the ability of PVE in restoring the capability of HFDPC to produce VEGF under stress, thus in promoting the hair growth. In other words, this result confirmed that PVE may be a potent anti-stress-induced hair growth ingredient.

2.4. Evaluation of the ability of PVE to protect hair growth from psychoemotional stress on human hair follicles

To evaluate the effect of PVE on the hair growth under cortisol stress, the following experiment was conducted.

Human hair follicles were removed from a fresh scalp/face skin biopsy of a 40 years old woman. After the depilation, isolated follicles were selected by microscopic observation to select only ones in anagen phase. They were randomized and placed in supplemented William's E medium (Penicillin/Streptomycin, L-glutamine (2mM), hydrocortisone (0.027μM), sodium selenite (10ng/ml), transferrin (10μg/ml) and insulin (10μg/ml) and indicated treatment (control, cortisol at 100 μM, PVE at 50 or 150 ppm in presence or not of cortisol).

Follicles were cultured at 37°C under 5% $CO_2$ and a relative humidity > 95%. Medium was changed at day 1 and 3. The growth of isolated follicles was monitored for 5 days by daily photography using a stereomicroscope device (Carl Zeiss, Microscopy, France). The length of each follicles was measured using ImageJ software.

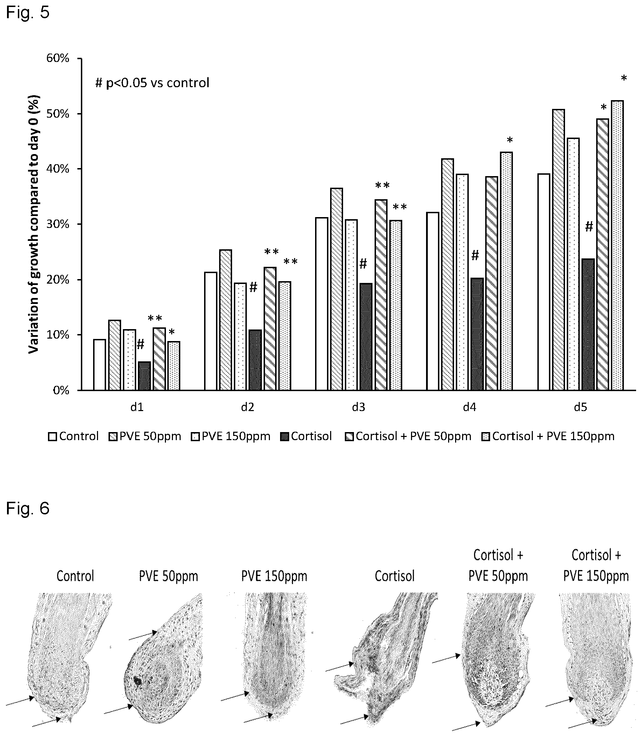

As a result, as shown in FIG. 5, when human hair follicles were treated with cortisol, the hair growth is strongly reduced from day 1 to day 5. It was observed that PVE counteracts the cortisol-induced slow-down hair growth. After 5 days, the growth of the stressed hair follicles treated with PVE is superior than the non-stressed hair follicles growth treated with PVE. In fact, the stressed hair follicles treated by PVE grew in average by 80 μm compared to the control condition. In parallel, it was observed that the length of the hair follicles in basal (non-stressed) condition is shorter than under cortisol treatment. Taken altogether, these results demonstrated the ability of PVE under stress condition, to restore and to promote afterwards the cortisol-induced hair growth.

2.5. Evaluation of the ability of PVE in the protection of the hair follicle structure

To evaluate the effect of PVE on the hair follicle structure under cortisol stress and the basal condition (non-stressed condition) the following experiment was conducted.

After overnight fixation in paraformaldehyde solution (MM France, Brignais, France), samples were dehydrated and impregnated in paraffin using a dehydration automat (STP120 ThermoScientific™ Waltham, MA, USA). Then, sections of 5 μM were mounted on Superfrost® Gold slides before proceeding to Hematoxylin-eosin-saffron (HES) staining.

Hematoxylin-eosin-saffron (HES) is a trichromatic coloration using nuclear, cytoplasmic and matrix colorants, usually used for the visualization of tissues.

As a result, as shown in FIG. 6, when human hair follicles were treated with cortisol, their structures (dermal sheath and hair matrix) are dramatically altered (highlighted by narrows). The dermal sheath is thinner. Conversely, the cortisol-stressed follicles treated by PVE harbor an intact structure and a conserved organization, notably of the dermal sheath layers. The dermal sheath is located at the outermost border of hair follicle and comprises the connective tissue sheath of the follicle. Dermal sheath cells are known to contribute to hair cycling and follicle neogenesis. Hair matrix is crucial for hair follicle life and especially for hair shaft production. Thus, preserving a strong dermal sheath and a viable hair matrix is also important for the maintenance of hair follicle homeostasis and for preventing the hair loss. The results showed that after 5 days in culture, the hair follicle is still viable (control condition), in contrast, after 5 days of cortisol treatment, the hair follicle is clearly damaged and fragilized. These results demonstrated the ability of PVE in protecting and reinforcing the hair follicles against negative effects of the cortisol but also in basal condition. Indeed, it was observed in basal condition (without stressor) in presence of PVE a thicker dermal sheath. In contrast, under cortisol condition, the dermal sheath is really thin, and the hair matrix seems to be altered.

2.6. Evaluation of the ability of PVE in preventing the cortisol-induced hair follicle cells apoptosis and in promoting their proliferation

To evaluate the effect of PVE on the hair follicle cells proliferation and apoptosis under cortisol stress, the following experiments were conducted.

To perform immunostaining, slide deparaffinization was manually conducted using successive baths of solvent. Heat-induced epitope retrieval was performed using the Dako Envision Flex solution (Agilent, Santa Clara, US). Ki-67 (dilution 1/500). Cell proliferation was studied by Ki-67 immunostainings realized on paraffin sections (mouse monoclonal anti-Ki-67,Dako™ M7240, Santa Clara, US), The apoptosis was studied by using click-iT® Plus Alexa 488 TUNEL assay (ThermoScientific™, C10617, Waltham, MA, USA) following supplier instructions.

After TUNEL assay and Ki67 antibody incubation, slices were incubated one hour at room temperature with an Alexa 568 coupled secondary antibody (A11004, Thermo Fischer Scientific™, Waltham, MA, USA). Nuclei counterstain was done using Hoechst (Invitrogen, ref H3570) at 1/5000 for 5 minutes at RT. Slides were mounted in aqueous medium.

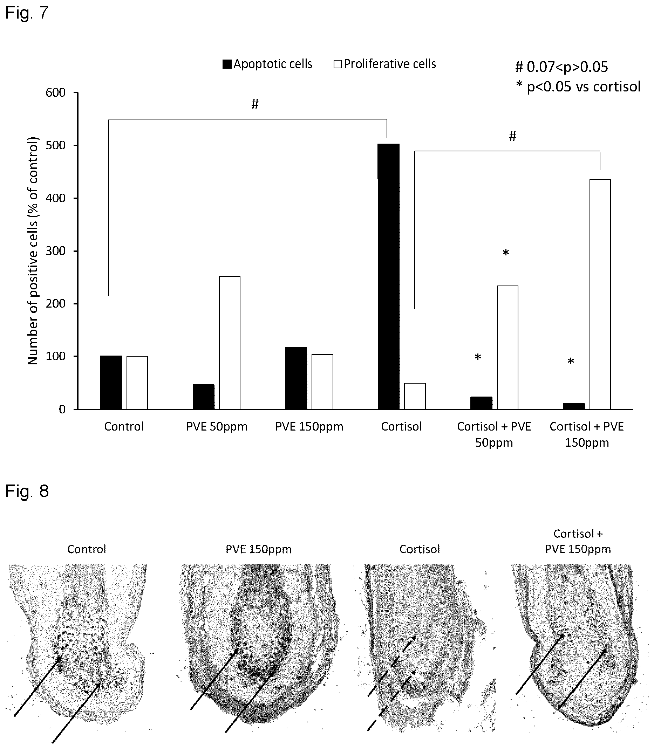

As a result, as shown in FIG. 7, a five days stress by cortisol stress induces apoptosis in cells, concomitantly with a reduced cell proliferation compared to control condition. This result demonstrated that the PVE counteracts efficiently the harmful effects of cortisol by significantly decreasing the number of apoptosis cells and strongly increases the hair follicle cell proliferation. This result confirmed the potent efficacy of PVE to counteract the cortisol-induced hair follicles damages, to avoid or delay the cortisol-induced hair loss, and to prevent a premature and prolonged resting phase, and to protect against hair fragility.

As expected, it was demonstrated, that psychoemotional stress mediated by cortisol release is responsible for a dramatic increase of apoptotic cells and decrease in cell viability. These data might explain the hair loss observed under psychoemotional stress. Interestingly, it was observed the ability of PVE in counteracting the cortisol-induced hair follicles cell damages apoptosis and decrease of cell proliferation.

Moreover, it was showed a dose dependent improving effect with PVE on apoptosis and cell proliferation.

2.7. Evaluation of the ability of PVE in the protection of the structure of hair follicles pigmentation

To evaluate the effect of PVE on the hair follicle pigmentation under basal or cortisol stress, the following experiments were conducted.

After overnight fixation in paraformaldehyde solution (MM France, Brignais, France), samples were dehydrated and impregnated in paraffin using a dehydration automat (STP120 ThermoScientific™ Waltham, MA, USA). The Then, sections of 5μM were mounted on Superfrost® Gold slides before proceeding to Masson's Fontana staining. This staining is used to visualize melanin granules. Melanin is a non-lipid pigment, brown to black, present physiologically in hair, eyes, and skin. As expected, it was demonstrated, that psychoemotional stress mediated by cortisol release is responsible for a dramatic increase of apoptotic cells and decrease in cell viability. These data might explain the hair loss observed under psychoemotional stress. Interestingly, it was observed the ability of PVE in counteracting the cortisol-induced hair follicles cell damages apoptosis and decrease of cell proliferation. Moreover, we showed a dose dependent improving effect with PVE on apoptosis and cell proliferation.

As a result, as shown in FIG. 8, when human hair follicles were treated with cortisol, the hair follicles pigmentation was highly impacted. Indeed, almost all the melanin granules disappeared after 5 days of treatment with cortisol.

Conversely, the melanin content of cortisol-stressed follicles treated by PVE was conserved. This result highlights the potential of PVE to alleviate the psychoemotional stress-induced hair greying. As expected, it was demonstrated that psychoemotional stress responsible for cortisol release is also responsible for hair greying. Indeed, it was shown less melanin (black arrows) in cortisol condition. Then, the results demonstrated the ability of PVE in preventing the cortisol-induced hair greying by promoting pigmentation. Furthermore, the pro-pigmenting effect of PVE was also observed in basal condition (at concentration of 150 ppm).

2.8. Evaluation of the ability of the PVE to counteract the glucocorticoids-induced increase of mast cells degranulation

To evaluate the effect of PVE on the mast cells degranulation, the following experiment was conducted.

The RBL-2H3 cell line is a commonly used beta hexosaminidase-releasing cell line used in inflammation, allergy and immunological research. This BL-2H3 cell line was used as a mast cells model and the beta-hexosaminidase was dosed as a marker of mast cell degranulation.

RBL-2H3, rat basophilic leukemia (ATCC, CRL-2256™) was maintained in DMEM, containing 10% FBS and 1% penicillin/streptomycin, at 37 °C, under 5% $CO_2$. Cells were seeded in 48-well plates, at $2.5 \times 10^5$ cells per well and incubate for 24 h.

After replacement with HEPES buffered Tyrode solution (pH 7.4), PVE was pretreated for 1 h, and then treated with 500 nM of A23187 a calcium ionophore inducer of cell degranulation. A23187 was used as an experimental positive control validating the experiment. The culture supernatant is recovered for 1 h after A23187 treatment. The cells are treated with Tyrode solution containing 1% triton X-100, incubated for 15 min, and then the supernatants were recovered by centrifugation. 50 μl of the culture supernatant and lysate were treated with 50 μl of p-nitrophenyl N-acetyl-beta-D-glucosaminide (4 mM), a beta-hexosaminidase substrate, respectively, and reacted at 37°C for 30 min. The reaction was stopped by treatment with 50 μl of 50 mM sodium carbonate buffer and measured at 405 nm.

Then, degranulation % was measured using the calculation of beta-hexosaminidase activity:

$$\text{Degranulation (\%)} = (OD_{supernatant} - OD_{blank}) / (OD_{supernatant} + OD_{lysate} - OD_{blank}) * 100$$

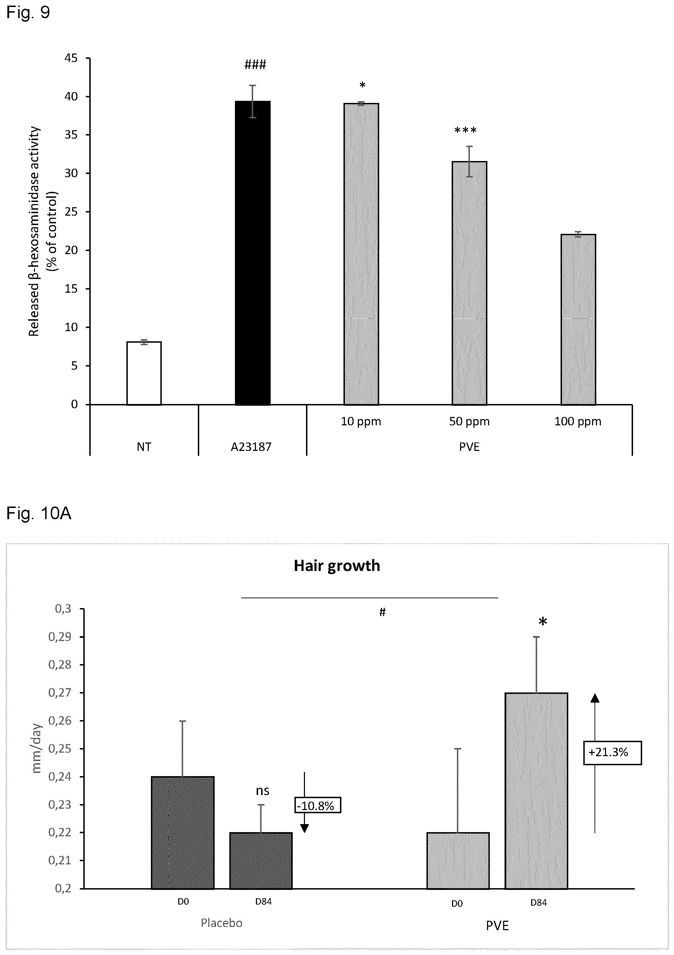

As shown in FIG.9, PVE inhibits mast cell degranulation in a dose dependent manner, thus suggesting its ability to mitigate the neurogenic inflammation responsible for hair loss and skin damages.

3. *In-vivo* study: Evaluation of the ability of PVE in preventing the stress-induced

hair loss and in enhancing the hair growth slow downed in stressed person To evaluate the effect of PVE on the hair follicles on human volunteers under cortisol stress, a double blind and vehicle controlled clinical study was performed. In this regard, the recommendations of the Declaration of Helsinki and the guidelines of the International Conference on Harmonization Good Clinical Practice were observed as applicable to a non-drug study.

To ensure that volunteers were stressed, their stress state was evaluated using the well-known Perceived Stress Scale (PSS) questionnaire and with a specific stress measurement device commercialized by the company Codesna (US-B2-10856797). PSS questionnaire is the most widely used tool in neuroscience field for measuring and rating the perception of human psycho-emotional stress. It consists to 10 questions with scored answers, leading to a global score as summarized below:

| PSS Scores | Stress perceived level |
|---|---|
| 0 to 13 | low |
| 14 to 26 | Moderate |
| 27 to 40 | high |

As it is agreed that a threshold of 24 could be reliable for anxiety detection, thus volunteers having scores above 24 were selected for the present study to evaluate the effect of PVE on hair loss and growth parameters.

To ensure that the selected volunteers are really stressed, a second selection by the means of the Codesna's device was performed. This measurement device is based on the studies performed on Brain-Heart interactions in neuro-cardiological field. Heart-rate variability is mirroring imbalances within the autonomic nervous system (sympathetic and parasympathetic responses) and directly reflects stress level. This device scores of the autonomic nervous system imbalance allowing to evaluate the effective chronic stress, regardless of any subjective (self-) evaluation. In fact, the device measures physiological parameters: the stress index, and the cardiorespiratory alignment that reflects the anxiety of a subject.

The following information is provided by the user's guide of the Codesna's device:

- Stress index:

  • < 15: non-stressed
  • 15 to 25: intermediate stressed
  • > 25: high stressed

- A cardiorespiratory alignment above 1.5 reflects an anxious state

The above selected volunteers were also passed through a third selection to ensure that they are suffering from stress-induced hair loss. In this regard, it is usual to perform a combing test consisting to comb volunteers in a standardized manner and to count the fall hair. In fact, only volunteers presenting more than 10 fall hair were considered as relevant for the rest of the study.

Importance of the measurement of the physiological stress level of volunteers:
27 women presenting a PSS score above 23 have been pre-selected. Their physiological stress was measured by using Codesna's device by following the user's guide.

Among the 27 volunteers, 13 women (representing 48%) presented a low physiological stress level under 13 (Table 1), meaning that these volunteers, although perceiving themselves as stressed, were not physiologically stressed as their organism managed it without physical repercussion.

Other 14 panelists presented a stress index above the threshold of 15, excepted for one who was at 14 but completed the other measured parameter: a cardiorespiratory alignment above 1.5 (1.64).

It can be concluded that these 14 volunteers were really stressed, and this justify their inclusion in the clinical study making their enrollment more relevant for the clinical evaluation of the effect of PVE.

Table 1: PSS scores and Stress index of non-included and included volunteers

| Non included volunteers | | | Included volunteers | | |
|---|---|---|---|---|---|
| Volunteer | PSS score | Stress index | Volunteer | PSS score | Stress index |
| Vol NonInc 1 | 29 | 9.8 | Vol Inc 1 | 40 | 22.6 |
| Vol NonInc 2 | 35 | 12.7 | Vol Inc 2 | 44 | 30.1 |
| Vol NonInc 5 | 36 | 2.6 | Vol Inc 3 | 35 | 29.3 |

(continued)

| Non included volunteers | | | Included volunteers | | |
|---|---|---|---|---|---|
| Volunteer | PSS score | Stress index | Volunteer | PSS score | Stress index |
| Vol NonInc 6 | 39 | 2.0 | Vol Inc 4 | 39 | 30.3 |
| Vol NonInc 8 | 36 | -9.7 | Vol Inc 6 | 37 | 23.7 |
| Vol NonInc 10 | 31 | 3.8 | Vol Inc 12 | 36 | 22.1 |
| Vol NonInc 13 | 23 | 9.5 | Vol Inc 14 | 38 | 32.5 |
| Vol NonInc 16 | 31 | 1.6 | Vol Inc 16 | 35 | 14.0 |
| Vol NonInc 17 | 27 | 2.5 | Vol Inc 18 | 36 | 17.3 |
| Vol NonInc 18 | 27 | 6.0 | Vol Inc 21 | 29 | 22.1 |
| Vol NonInc 19 | 31 | 10.2 | Vol Inc 27 | 38 | 33.1 |
| Vol NonInc 20 | 31 | 9.4 | Vol Inc 29 | 45 | 45.7 |
| Vol NonInc 21 | 35 | -3.9 | Vol Inc 30 | 24 | 29.3 |
| | | | Vol Inc 34 | 34 | 19.6 |

3.2. Evaluation of the ability of PVE in preventing the stress-induced hair loss and in enhancing the hair growth slow downed in stressed volunteers

As shown in the table 1 above, fourteen Caucasian women (aged between 27 and 56, mean age 42.6) were selected as being stressed and suffering from stress related hair damages (hair loss, slow down hair growth rate). The question is to know whether such hair loss is at least partly due to their stress.

Then, the volunteers were asked to apply on their scalp a gel cream formulation comprising either PVE or a placebo (Table 2) once a day and for 84 days. Before the treatment, meaning 48h prior to the first application and 48h before the 84 days- treatment visit, 1cm$^2$ scalp area was shaved for Trichoscan analysis. Pictures were taken at Day 0 - 48h and Day 0 as well as at Day 84 - 48h and Day 84 to evaluate the hair growth (measure of hair length), the proportion of anagen phase, the proportion of telogen phase, and the anagen/telogen ratio.

Table 2: Gel cream formulation comprising PVE used in the clinical study

| Gel cream composition | Placebo (%) | Composition with PVE (%) |
|---|---|---|
| Water | 91.45 | 89.45 |
| Aristoflex® Silk (Sodium polyacryloyldimethyl taurate) | 0.2 | 0.2 |
| Buffer solution (Citric acid and sodium citrate) | 1 | 1 |
| Nipaguard® EHP (Phenoxyethanol and ethylhexylglycerin) | 0.8 | 0.8 |
| PVE | 0 | 2 |
| Hostacerin® L20 (polysorbate 20) | 1.5 | 1.5 |
| Ethanol (99%) | 5 | 5 |
| Fragrance | 0.05 | 0.05 |

The two gel creams summarized in table 2 were applied on the scalp of the selected stressed volunteers in table 1.

The length of the hair, the percentage of the hair follicles in anagen phase, the percentage of the hair follicles in telogen phase, and the anagen/telogen ratio were measured by pictures analysis of a shaved area, immediately after shaving or 48h hours after, using Trichoscan. It was done before the use of the gel creams and 84 days after. Statistics: **: $p < 0.01$, *: $p < 0.05$, #: $p < 0.1$, ns: non-significant

As shown in Fig. 10A, PVE enhances the hair growth in stressed volunteers. Indeed, firstly, it demonstrates that the placebo did not counteract the stress-induced slow down hair growth. Indeed, the hair growth rate decreased by 10,8% at D84. This result demonstrates that the selected volunteers were really stressed as their hair growth rate decreased over time. It is consistent with the *in vitro* results as well (Fig. 2 to 8). Secondly, it demonstrates that the hair grew up to 21,3% compared to the control after 84 days of treatment. Therefore, it can be concluded that PVE inherently counteracts the harmful effect of the stress on the hair and also enhances the hair growth rate slow downed by stress.

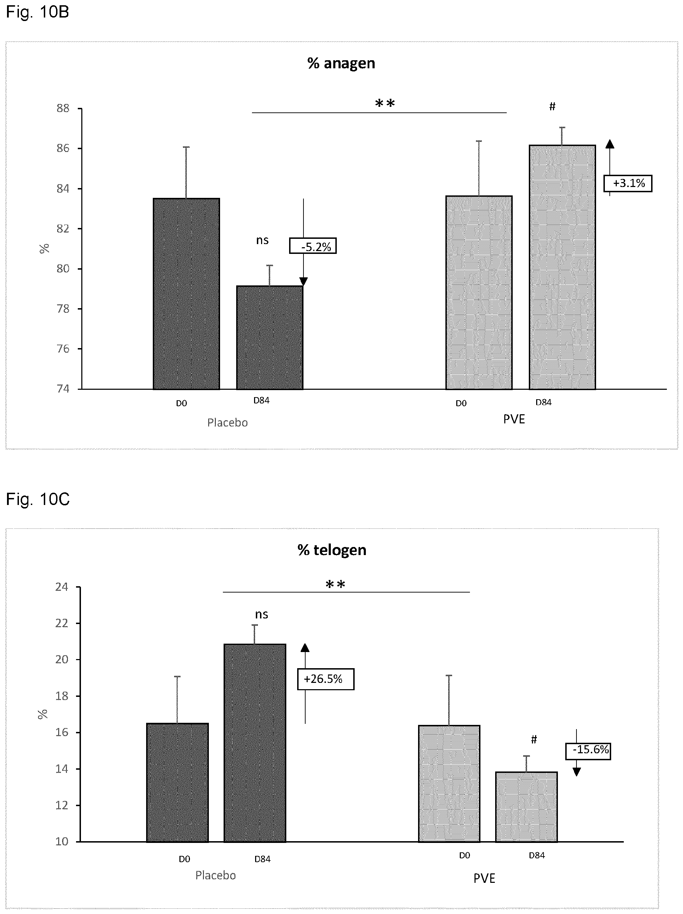

As shown in Fig. 10B, as the proportion of hair follicles in anagen phase decreased by 5,2% compared to the control, it demonstrates that the placebo did not manage to maintain the hair follicles in anagen phase after 84 days of treatment. In contrary, PVE increases the anagen phase proportion (+ 3,1%) meaning that hair is in growth phase despite the stress. These statements are consistent with the *in vitro* results as well (e.g: Fig. 3). Therefore, PVE prevents the stress-induced hair loss.

As shown in Fig. 10C, as the proportion of hair follicles in telogen phase increased by 26,5% at D84 compared to the control, it demonstrates that the placebo did not manage to counteract the entrance of the hair follicles into telogen phase. In contrary, PVE decreases the telogen phase proportion by 15,6% at D84 compared to the control, meaning that hair is maintaining in resting phase and does not fall. These statements are also consistent with the *in vitro* results (Fig. 2 to 8). Therefore, PVE prevents the stress-induced hair loss.

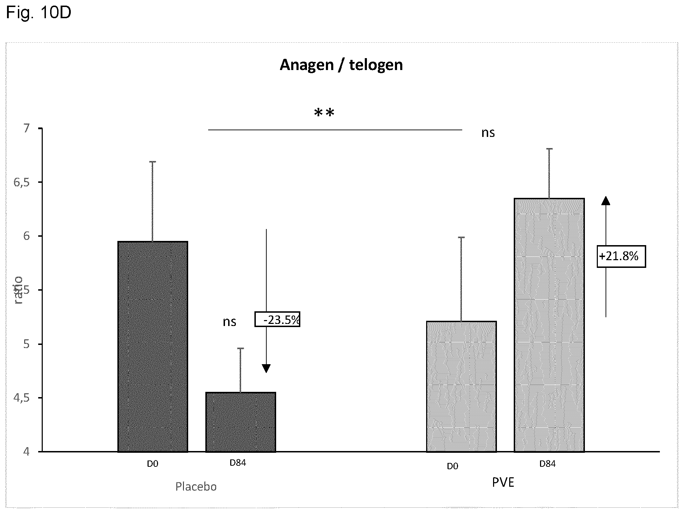

As shown in Fig. 10D, as the anagen/telogen ratio decreased by 23,5% at D84, it demonstrates that the placebo did not manage to counteract the stress-induced entrance of hair follicles into the telogen phase. In contrary, PVE enhances the stress-induced anagen/telogen ratio in a significant manner. These statements are consistent with the *in vitro* results (Fig. 2 to 8). Therefore, it reflects that PVE promotes the hair dynamic and its development by stimulating the growth phase (anagen) entrance and by inhibiting the resting phase (telogen) entrance, which is ultimately apparent on the hair growth speed and prevents the stress-induced hair loss.

**Claims**

1. An extract of the aerial parts of *Prunella vulgaris* **characterized in that**:

 i) it is an aqueous and/or a hydroalcoholic extract of the leaves, stems and flowers;
 ii) it comprises 1 to 15%, preferably 2 to 12%, more preferably 3 to 10% by weight of one or more polyphenols, based on the total weight of the dry content of the extract;
 iii) it comprises less than 0,1%, preferably less than 0,01%, more preferably less than 0,001%, particularly 0% by weight of saponins, based on the total weight of the dry content of the extract.

2. An extract according to claim 1, wherein the extract is an aqueous extract.

3. An extract according to claim 1, wherein the hydroalcoholic extract is a hydromethanolic and/or a hydroethanolic extract.

4. A method for preparing an extract of the leaves, stems and flowers of *Prunella vulgaris* comprising the steps of:

 i) conducting the extraction of the said aerial parts with a water or a hydroalcoholic solvent, wherein:

 a. the water extraction is being performed at 80 to 150°C, preferably at 100 to 130°C, even more preferably at 85 to 130°C, particularly at 90 to 120°C for 15 minutes to 10 hours, preferably for 30 minutes to 8 hours, more preferably for 1 hour to 6 hours, most preferably for 1.5 to 5 hours; or
 b. the hydroalcoholic extraction is conducted at 50 to 120°C, preferably at 60 to 115°C, more preferably at 70 to 110°C, particularly at 80 to 105°C for 15 minutes to 10 hours, preferably for 30 minutes to 8 hours, more preferably for 1 hour to 6 hours, most preferably for 1.5 to 5 hours;

 (ii) separating the extract from the solid residuals of step (i); and
 (iv) optionally concentrating the extract by removing the at least one solvent or parts thereof.

5. An extract of the aerial parts of *Prunella vulgaris* obtainable by implementation of the preparation method according to claim 4.

6. A cosmetic or pharmaceutic composition comprising:

 (A) an extract of the aerial parts of *Prunella vulgaris* according to any of the claims 1 to 3 and 5, as an active ingredient; and
 (B) at least one further cosmetically and/or pharmaceutically acceptable ingredient other than the extract of *Prunella vulgaris,*

wherein the composition is a composition for topic use selected from the group consisting of a solution, a suspension, an emulsion, a cream, a paste, a gel, a lotion, a powder, a soap, a surfactant-containing water, an oil, a shampooing, and a spray; and/or wherein the composition is a nutraceutical composition which is administered orally.

7. The cosmetic or pharmaceutic composition according to claim 6, wherein the composition comprises 0.0001 to 20% by weight, preferably 0.001 to 15% by weight, more preferably 0.005 to 10% by weight, in particular 0.01 to 5% by weight, referred to the total weight of the composition, of an extract of the aerial parts of *Prunella vulgaris* according to any of the claims 1 to 3 and 5.

8. Cosmetic use of a composition comprising or consisting of an extract of the aerial parts of *Prunella vulgaris* according to any of the claims 1 to 3 and 5, as an active ingredient intended for preventing and/or treating the psychoemotional stress related disorders and/or damages on the skin and the hair follicles.

9. The cosmetic use according to claim 8, wherein said extract is for regulating the stress-induced neuro-immune excitation, for regulating the stress-induced sensory neuron excitation, for counteracting the stress-induced mast cell degranulation, for promoting the secretion of at least one well-being hormone, or two or more thereof.

10. The cosmetic use according to any of the claim 9, wherein the well-being hormone is selected from the beta-endorphin, the oxytocin, the serotonin, the dopamine, or a combination of two or more thereof.

11. The cosmetic use according to any of the claims 8 to 10, wherein the said extract has at least one of the following activities on the skin cells and/or the hair follicles:

   (a) counteracting and/or reducing the stress-induced degranulation of mast cells;
   (b) preventing the skin stress-induced inflammation and/or irritation;
   (c) alleviating or counteracting the cortisol-induced hair growth;
   (d) restoring the stressed hair follicles in producing VEGF;
   (e) alleviating or counteracting the cortisol-induced hair loss;
   (f) counteracting the cortisol-induced hair follicles damages;
   (g) promoting cell proliferation reduced in hair follicles exposed to cortisol;
   (h) counteracting the cortisol-induced hair follicles apoptosis;
   (i) preventing or limiting the cortisol-induced hair greying;
   (j) promoting the hair densification reduced by the cortisol;
   (k) preventing or limiting the cortisol induced hair thinning;
   (l) a combination of two of more thereof.

12. The use according to any of the claims 8 to 11, wherein the stress-related disorder and/or damage is selected from skin dryness, skin redness, skin aberrant pigmentation, sebum-deregulation, skin itching, skin inflammation, atopic dermatitis, psoriasis, vitiligo, systemic lupus erythematosus, hair loss, hair growth arrest, hair follicles apoptosis, hair thickness, hair greying and hair thinning, or two or more thereof.

13. The use according to any of the claims 8 to 12, wherein the stress-related disorders are hair loss, hair growth arrest, hair follicles apoptosis, hair thickness, hair greying and hair thinning.

14. A cosmetic method intended for preventing and/or for treating the stress-induced disorders and/or damages in the skin and in the hair follicles of a subject by applying, onto the skin or the scalp on need, an effective amount of a composition comprising or consisting of an extract of the aerial parts of *Prunella vulgaris* according to any of the claims 1 to 3, 5 to 7.

15. A composition comprising or consisting of an extract of the aerial parts of *Prunella vulgaris* according to any of claims 1 to 3, 5, as an active ingredient for use in a method for preventing and/or for treating the stress-induced disorders and/or damages in the skin and in the hair follicles.

Fig. 1A

Fig. 1B

Fig. 1C

Fig. 1D

Fig. 2A

Fig. 2B

Fig. 3

Fig. 4

Fig. 5

# p<0.05 vs control

Variation of growth compared to day 0 (%)

□ Control  ▨ PVE 50ppm  ▨ PVE 150ppm  ▨ Cortisol  ▨ Cortisol + PVE 50ppm  ▨ Cortisol + PVE 150ppm

Fig. 6

| Control | PVE 50ppm | PVE 150ppm | Cortisol | Cortisol + PVE 50ppm | Cortisol + PVE 150ppm |

Fig. 7

Fig. 8

Fig. 9

Fig. 10A

Fig. 10B

% anagen

Fig. 10C

% telogen

Fig. 10D

Anagen / telogen

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 5 624 673 A (BONTE FREDERIC [FR] ET AL) 29 April 1997 (1997-04-29)<br><br>* page 1, lines 5-20 *<br>* page 2, lines 18-46; example 12 * | 1,2, 4-11,14, 15 | INV.<br>A61K8/9789<br>A61K8/9783<br>A61Q19/00<br>A61Q7/00<br>A61P17/14 |
| X | CN 106 727 928 A (UNIV TIANJIN) 31 May 2017 (2017-05-31)<br>* paragraphs [0002], [0087]; claims 1,2 * | 1,3,6,7 | A61K31/00<br>A61K8/34<br>A61K8/36 |
| A | KR 2018 0045109 A (BIO SPECTRUM INC [KR]) 4 May 2018 (2018-05-04)<br>* abstract * | 1-15 | |
| A | ALEXOPOULOS ALEX ET AL: "Stress-related skin disorders",<br>REVIEWS IN ENDOCRINE & METABOLIC DISORDERS, NEW YORK, NY : SPRINGER, US, vol. 17, no. 3, 2 July 2016 (2016-07-02), pages 295-304, XP036116389, ISSN: 1389-9155, DOI: 10.1007/S11154-016-9367-Y [retrieved on 2016-07-02]<br>* the whole document * | 1-15 | |
| X | CN 108 272 858 B (CHENGDU MEDICAL COLLEGE) 7 May 2021 (2021-05-07)<br>* paragraph [0047]; claims 1-6; example 1; table 1 * | 1,3-7 | TECHNICAL FIELDS SEARCHED (IPC)<br><br>A61K<br>A61Q<br>A61P |

The present search report has been drawn up for all claims

| Place of search | Date of completion of the search | Examiner |
|---|---|---|
| Munich | 17 February 2022 | Verrucci, Marinella |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 30 6215

This annex lists the patent family members relating to the patent documents cited in the above-mentioned European search report.
The members are as contained in the European Patent Office EDP file on
The European Patent Office is in no way liable for these particulars which are merely given for the purpose of information.

17-02-2022

| Patent document cited in search report | | Publication date | Patent family member(s) | | Publication date |
|---|---|---|---|---|---|
| US 5624673 | A | 29-04-1997 | EP | 0589934 A1 | 06-04-1994 |
| | | | FR | 2677248 A1 | 11-12-1992 |
| | | | JP | H06507892 A | 08-09-1994 |
| | | | US | 5624673 A | 29-04-1997 |
| | | | WO | 9221322 A1 | 10-12-1992 |
| CN 106727928 | A | 31-05-2017 | NONE | | |
| KR 20180045109 | A | 04-05-2018 | NONE | | |
| CN 108272858 | B | 07-05-2021 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6293217 A
- US 5624673 A
- JP 0710722 A
- KR 101864720 B
- EP 3681467 A
- EP 3643295 A
- US 10856797 B2

**Non-patent literature cited in the description**

- **PAUS et al.** *Review CellPress,* 2014, vol. 20 (10), 559-570
- **TOPS M. et al.** *Frontiers in Psychiatry,* 2012, vol. 3
- **X-J GU et al.** *Helvetica Chimica Acta,* 2007, vol. 90, 72-78
- **K-B ROH.** *Research Article,* 2018